# EUROPÄISCHE PATENTSCHRIFT

(11) **EP 0 673 932 B1**
(45) Veröffentlichungstag und Bekanntmachung des Hinweises auf die Patenterteilung: **07.06.2000**
(21) Anmeldenummer: 95103582.3
(22) Anmeldetag: 13.03.1995
(51) Int. Cl.: C07D 213/82, C07D 213/89, A61K 31/44

(54) **Sulfonamidocarbonylpyridin-2-carbonsäureesteramide sowie ihre Pyridin-N-oxide, Verfahren zu ihrer Herstellung und ihre Verwendung als Arzneimittel**
Sulfonamidocarbonyl-pyridine-2-carboxamides as well as their pyridine-N-oxides, process for their preparation and their use as medicaments
Sulfonamidocarbonyl-pyridine-2-carboxamides ainsi que leurs pyridine-N-oxydes, procédé pour leur préparation et leur utilisation comme médicaments

(30) Priorität: 25.03.1994 DE 4410480
(43) Veröffentlichungstag der Anmeldung: 27.09.1995
(73) Patentinhaber: HOECHST AKTIENGESELLSCHAFT, 65929 Frankfurt am Main (DE)
(72) Erfinder: Weidmann, Klaus, Dr., D-61476 Kronberg (DE); Bickel, Martin, Dr., D-61348 Bad Homburg (DE); Günzler-Pukall, Volkmar, D-35039 Marburg (DE)

(56) Entgegenhaltungen:
- EP-A- 0 541 042
- EP-A- 0 562 512

## Beschreibung

Die Erfindung betrifft Sulfonamidocarbonylpyridin-2-carbonsäureesteramide und ihre Pyridin-N-oxide sowie ihre Verwendung als Arzneimittel gegen fibrotische Erkrankungen.

Verbindungen, die die Enzyme Prolyl- und Lysylhydroxylase inhibieren, bewirken eine sehr selektive Hemmung der Kollagenbiosynthese durch Beeinflussung der kollagenspezifischen Hydroxylierungsreaktionen. In deren Verlauf wird proteingebundenes Prolin oder Lysin durch die Enzyme Prolyl- bzw. Lysylhydroxylase hydroxyliert. Wird diese Reaktion durch Inhibitoren unterbunden, so entsteht ein nicht funktionsfähiges, unterhydroxyliertes Kollagenmolekül, das von den Zellen nur in geringer Menge in den extrazallulären Raum abgegeben werden kann. Das unterhydroxylierte Kollagen kann außerdem nicht in die Kollagenmatrix eingebaut werden und wird sehr leicht proteolytisch abgebaut. Als Folge dieser Effekte verringert sich insgesamt die Menge des extrazellulär abgelagerten Kollagens.

Inhibitoren der Prolylhydroxylase sind deshalb geeignete Substanzen in der Therapie von Erkrankungen, in denen die Ablagerung von Kollagenen maßgeblich zum Krankheitsbild beiträgt. Hierzu gehören u. a. Fibrosen der Lunge, Leber und Haut (Skleroderma) sowie die Atherosklerose.

Es ist bekannt, daß das Enzym Prolylhydroxylase durch Pyridin-2,4- und - 2,5-dicarbonsäure effektiv gehemmt wird (K. Majamaa et al., Eur. J. Biochem. 138 (1984) 239-245). Diese Verbindungen sind in der Zellkultur allerdings nur in sehr hohen Konzentrationen als Hemmstoffe wirksam (Tschank, G. et al., Biochem. J. 238 (1987) 625-633).

In der DE-A 34 32 094 werden Pyridin-2,4- und -2,5-dicarbonsäurediester mit 1-6 C-Atomen im Esteralkylteil als Arzneimittel zur Inhibierung der Prolyl- und Lysylhydroxylase beschrieben.

Diese niedrig-alkylierten Diester haben jedoch den Nachteil, daß sie zu schnell im Organismus zu den Säuren gespalten werden und nicht in genügend hoher Konzentration an ihren Wirkort in der Zelle gelangen und damit für eine eventuelle Verabreichung als Arzneimittel weniger geeignet sind.

Die DE-A 37 03 959, DE-A 37 03 962 und DE-A 37 03 963 beschreiben in allgemeiner Form gemischte Ester/Amide, höher alkylierte Diester und Diamide der Pyridin-2,4- und -2,5-dicarbonsäure, die die Kollagenbiosynthese im Tiermodell wirksam hemmen.

In der EP-A-0 541 042 sind unsubstituierte Pyridin-2,4- und -2,5-dicarbonsäurediamide beschrieben, die die Prolylhydroxylase hemmen. In der EP-A-0 562 512 sind Verbindungen beschrieben, die aufgrund der Hemmung der Prolylhydroxylase antifibrotische Wirkung haben.

Die Verbindungen der Formel I sind Ester-Prodrugs und werden im lebenden Organismus (in vivo) und in Zellkulturen (invitro) in die entsprechenden freien Carbonsäuren gespalten.

In der gleichzeitig eingereichten deutschen Anmeldung P 44 10 423.5 sind die entsprechenden Carbonsäuren der Prodrugs der Formel I beschrieben.

Es bestand nunmehr die Aufgabe nach Verbindungen zu suchen, die noch stärker die Prolylhydroxylase hemmen als die bisher bekannten Verbindungen und damit zu einer stärkeren Hemmung der Kollagenbiosynthese führen.

Gelöst wird die Aufgabe durch das Bereitstellen von Sulfonamidocarbonylpyridin-2-carbonsäureesteramiden sowie ihrer Pyridin-N-oxide der allgemeinen Formel I 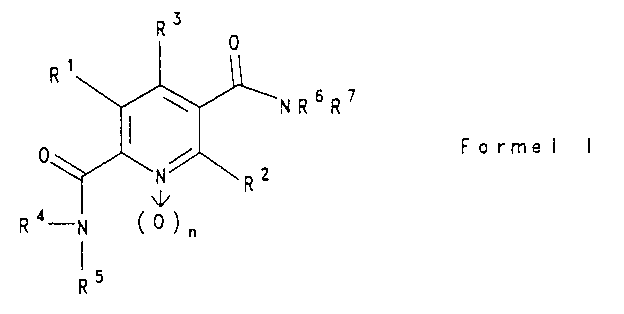 worin
- R¹: Hydroxy, (C₁-C₆)-Alkoxy,
- R² und R³: Wasserstoff,
- R⁶: Wasserstoff oder ein 1 oder 2-wertiges physiologisch verwendbares Kation, insbesondere Na⊕, K⊕, Mg²⊕ oder Ca²⊕ oder ein Ammoniumion, insbesondere H₃N⊕C(CH₂OH)₃,
- R⁷: einen Rest der Formel II, ausgenommen -SO₂H, bedeutet

-Y-[C-U]ᵣ-D-W (II),

in welchem
- Y: -SO₂-,
- C: eine Bindung oder
(C₁-C₁₆)-Alkandiyl,
- U: eine Bindung,
- r: 1 ist,
- D: eine Bindung oder Wasserstoff
- W: einen Phenylrest bedeutet, wobei W einfach oder zweifach substituiert sein kann, ist durch Fluor, Chlor, (C₁-C₆)-Alkyl, (C₁-C₆)-Alkoxy,
und W zusätzlich einfach substituiert ist durch Phenyl, Phenoxy, -O-[CH₂]_{X}C_{f}H_{(2f + 1-g)}F_{g}, Carbamoyl, N-(C₁-C₁₀)-Alkylcarbamoyl, N,N-Di-(C₁-C₈)-alkylcarbamoyl, N-(C₃-C₈)-Cycloalkylcarbamoyl, N-Phenylcarbamoyl, N-(C₇-C₁₁)-Phenylalkylcarbamoyl, N-((C₁-C₄)-Alkoxy-(C₁-C₄)alkyl)-carbamoyl, N-Phenoxy-(C₁-C₄)alkyl)-carbamoyl,
(C₁-C₁₀)-Alkanoylamino-(C₁-C₂)-alkyl, (C₃-C₈)-Cycloalkanoylamino-(C₁-C₂)-alkyl, Benzoylamino-(C₁-C₂)-alkyl oder (C₇-C₁₁)-Phenylalkanoylamino-(C₁-C₂)-alkyl,
wobei die Reste, die einen Arylrest enthalten, ihrerseits am Aryl substituiert sein können mit 1, 2, 3, 4 oder 5 gleichen oder verschiedenen Substituenten aus der Reihe Hydroxy, (C₁-C₄)-Alkoxy, Phenoxy, Benzyloxy, Fluor, Chlor, Trifluormethyl,
Carbamoyl, N-(C₁-C₆)-Alkylcarbamoyl, N,N-Di-(C₁-C₆)-alkylcarbamoyl, N-(C₃-C₈)-Cycloalkylcarbamoyl, N-Phenylcarbamoyl, N-(C₇-C₁₁)-Phenalkylcarbamoyl,
- R⁴: Wasserstoff,
- R⁵: eine Methyl-Gruppe bedeutet, die mit (C₁-C₁₂)-Alkoxycarbonyl, (C₅-C₆)-Cycloalkoxycarbonyl oder Benzyloxycarbonyl substituiert ist, und
- n: 0,
- f: 1 bis 5,
- g: 0,1 bis (2f + 1) und
- x: 0 oder 1
bedeuten.

Bevorzugt sind Verbindungen der allgemeinen Formel I, in der
- R¹: Hydroxy, (C₁-C₄)-Alkoxy,
- R² und R³: Wasserstoff,
- R⁶: Wasserstoff oder ein 1 oder 2-wertiges physiologisch verwendbares Kation, insbesondere Na⊕, K⊕, Mg²⊕ oder Ca²⊕ oder ein Ammoniumion, insbesondere H₃N⊕C(CH₂OH)₃,
- R⁷: einen Rest der Formel II, ausgenommen -SO₂H, bedeutet

-Y-[C-U]ᵣ-D-W (II),

in welchem
- Y: -SO₂-,
- C: eine Bindung oder
(C₁-C₁₆)-Alkandiyl,
- U: eine Bindung,
- r: 1 ist,
- D: eine Bindung oder Wasserstoff
- W: einen Phenylrest bedeutet, wobei W einfach oder zweifach substituiert ist durch N-(C₃-C₈)-Cyclohexylcarbamoyl,
- R⁴: Wasserstoff,
R⁵ eine Methyl-Gruppe bedeutet, die mit (C₁-C₈)-Alkoxycarbonyl, (C₅-C₆)-Cycloalkoxycarbonyl oder Benzyloxycarbonyl substituiert ist, und
- n: 0,
- f: 1 bis 5,
- g: 0,1 bis (2f+1) und
- x: 0 oder 1
bedeuten.

Weiterhin betrifft die Erfindung Verbindungen der allgemeinen Formel I sowie die physiologisch verträglichen Salze zur Behandlung von Störungen des Stoffwechsels von Kollagen und kollagenähnlichen Stoffen sowie von fibrotischen Erkrankungen.

Schließlich betrifft die Erfindung ein Arzneimittel, enthaltend mindestens eine Verbindung der allgemeinen Formel I und gegebenenfalls einen pharmazeutisch verträglichen Träger.

Insbesondere betrifft die Erfindung Verbindungen der Formel I zur Anwendung als Inhibitor der Prolylhydroxylasehemmer in vivo und in vitro und als Fibrosuppressiva.

Weiterhin betrifft die Erfindung ein Verfahren zur Herstellung von Verbindungen der allgemeinen Formel I.

Verbindungen der Formel I, in denen
- Y: = SO₂ bedeutet,
wurden hergestellt, indem
i) die Pyridin-2-carbonsäure-Derivate bzw. die entsprechenden Ester der Formel 11 mit den Aminen der Formel 5 umgesetzt wurden, oder
ii) die Pyridin-5-carbonsäure-Derivate der Formel 12 mit den Sulfonamid-Derivaten der Formel 9 umgesetzt wurden, oder
iii) die Pyridin-5-carbonsäureamid-Derivate der Formel 13 mit den Sulfonsäure-Derivaten der Formel 2 umgesetzt wurden, vgl. Schema 2,
wobei die Verbindungen der Formeln 12 bzw. 13 ihrerseits aus den Verbindungen der Formel 7 nach den bekannten Methoden hergestellt wurden.

Schema 1 verdeutlicht die Herstellung von Verbindungen der Formel Ia oder Ib, in denen Y = SO₂ bedeutet:

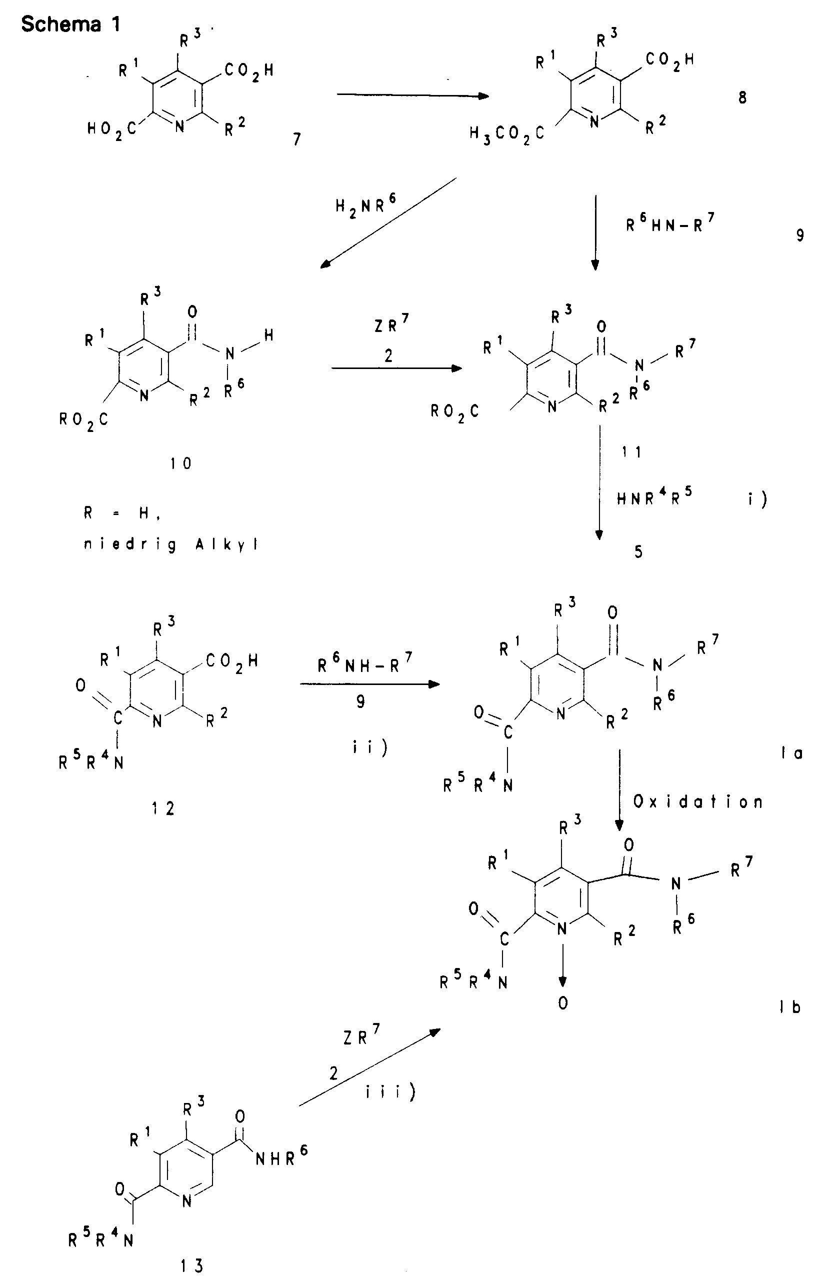

Entsprechend CA: Vol 68, 1968, 68840 h können aus den substituierten Pyridin-2,5-dicarbonsäuren der Formel 7 unter Veresterungsbedingungen die Pyridin-2-carbonsäureester-5-carbonsäuren der Formel 8 hergestellt werden. Geeignete Bedingungen sind z.B. die Veresterung mit Methanol in Gegenwart von Schwefelsäure, wobei die Reaktionszeit so zu wählen ist, daß die vollständige Veresterung zum Diesterprodukt nur untergeordnet stattfindet, bzw. die Diesterprodukte als Nebenprodukte abgetrennt werden können.

Die Herstellung der Verbindungen der Formel 11 erfolgt aus den Verbindungen der Formel 8 und den Sulfonamidderivaten der Formel 9 (Y = SO₂), wobei es zweckmäßig sein kann, beide Reaktanden mit Hilfsreagenzien zu aktivieren (Houben-Weyl: Methoden der Organischen Chemie, Band IX, Kapitel 19, Seiten 636-637).

An Reagenzien zur Carbonsäurereaktivierung können die dem Fachmann bekannten Substanzen, wie Thionylchlorid, Oxalylchlorid, Pivaloylchlorid oder Chlorameisensäureester-Derivate Verwendung finden. Es ist nicht immer notwendig, diese aktivierten Derivate der Verbindungen der Formel 8 zu isolieren. Meist ist es zweckmäßig, sie nach Herstellung in situ oder als Rohprodukt mit den Sulfonamidderivaten der Formel 9 umzusetzen.

Zweckmäßigerweise werden die Verbindungen der Formel 9 zunächst mit einer anorganischen oder organischen Base, wie z.B. Natrium- oder Kaliumhydroxid, -carbonat, -alkoxid, -hydrid, -amid, Ammoniak, Triethylamin, Tributylamin, Pyridin bei -20° bis + 150° C, vorzugsweise bei 0° - 80° C zur Reaktion gebracht und dieses Reaktionsgemisch mit einer Verbindung der Formel 8 oder dessen aktivierter Form umgesetzt. Die Umsetzung erfolgt in einem inerten Lösungsmittel, wie
z.B. Methylenchlorid, Methanol, Ethanol, Aceton, Essigsäureethylester, Toluol, Tetrahydrofuran, Acetonitril, N,N-Dimethylformamid, N,N-Dimethylacetamid, Nitromethan, Dimethylsulfoxid oder Gemischen dieser Lösungsmittel. Alternativ können die Ester der Formel 11 mit Hilfe der üblichen Kondensationsreagenzien (wie z.B. N,N'-Dicyclohexylcarbodiimid/4-N,N-Dimethylaminopyridin) hergestellt werden.

Die Reaktion der Pyridin-2-carbonsäureester 11 mit Aminen HNR⁴R⁵ führt zu den erfindungsgemäßen Verbindungen der Formel Ia.

Alternativ können zur Herstellung der Verbindungen der Formel Ia die Verbindungen 11 (R = niederes Alkyl) zu den Pyridin-2-carbonsäure-Derivaten 11 (R = H) verseift werden und diese anschließend mit den Aminen HNR⁴R⁵ nach den üblichen Methoden der Peptidchemie zu den erfindungsgemäßen Verbindungen der Formel Ia gekuppelt werden.

Die weitere Umsetzung zu den Pyridin-N-oxiden der Formel Ib erfolgt durch Oxidation von Verbindungen der Formel Ia.

Eine allgemeine Vorschrift dieser Oxidationsmethode ist auch beispielsweise in "E. Klinsberg, Pyridine and its Derivatives, Interscience Publishers, New York, 1961, Part 2, 93" beschrieben.

Die Oxidation mit Wasserstoffperoxid ist beispielsweise in "E. Ochiai, J. Org. Chem. 18, 534 (1953)" beschrieben.

Die Verfahrensbedingungen können im Detail der deutschen Patentanmeldung P 38 26 471.4, 38 28 140.6, 39 24 093.2, 40 01 002.3 sowie den DE-A-37 03 959, 37 03 962 und 37 03 963 entnommen werden.

Zur Herstellung der Verbindungen der Formel I, in denen NR⁴R⁵ einen Alkoxycarbonylmethyl- oder einen Benzyloxycarbonylmethyl-Rest (NR⁴R⁵ = NH CH₂ CO₂R) bedeutet, wurden die Verbindungen der Formel 11 (R = niederes Alkyl) zu den Pyridin-2-carbonsäure-Derivaten der Formeln 11 (R = H) verseift und diese mit den entsprechenden Glycinesterderivaten kondensiert.

Zur Herstellung von Verbindungen gemäß der allgemeinen Formel I (Ia, Ib) nach dem Schema 1 werden Verbindungen eingesetzt, in denen R⁶ Wasserstoff bedeutet. Die Salzbildung, nach der R⁶ ein physiologisch verwendbares Kation bedeutet, erfolgt bevorzugt anschließend. Als Salzbildner kommen bevorzugt N-Alkylamine, (Hydroxyalkyl)amine und (Alkoxyalkyl)amine, wie z.B. 2-Ethanolamin, 3-Propanolamin, 2-Methoxyethylamin, 2-Ethoxyethylamin und α,α,α-Tris-(hydroxymethyl)methylamin (= Trispuffer, Tromethan) oder auch basische Aminosäuren, wie z.B. Histidin, Arginin und Lysin in Frage.

Die Einführung des Substituenten R¹ wird in Schema 2 verdeutlicht.

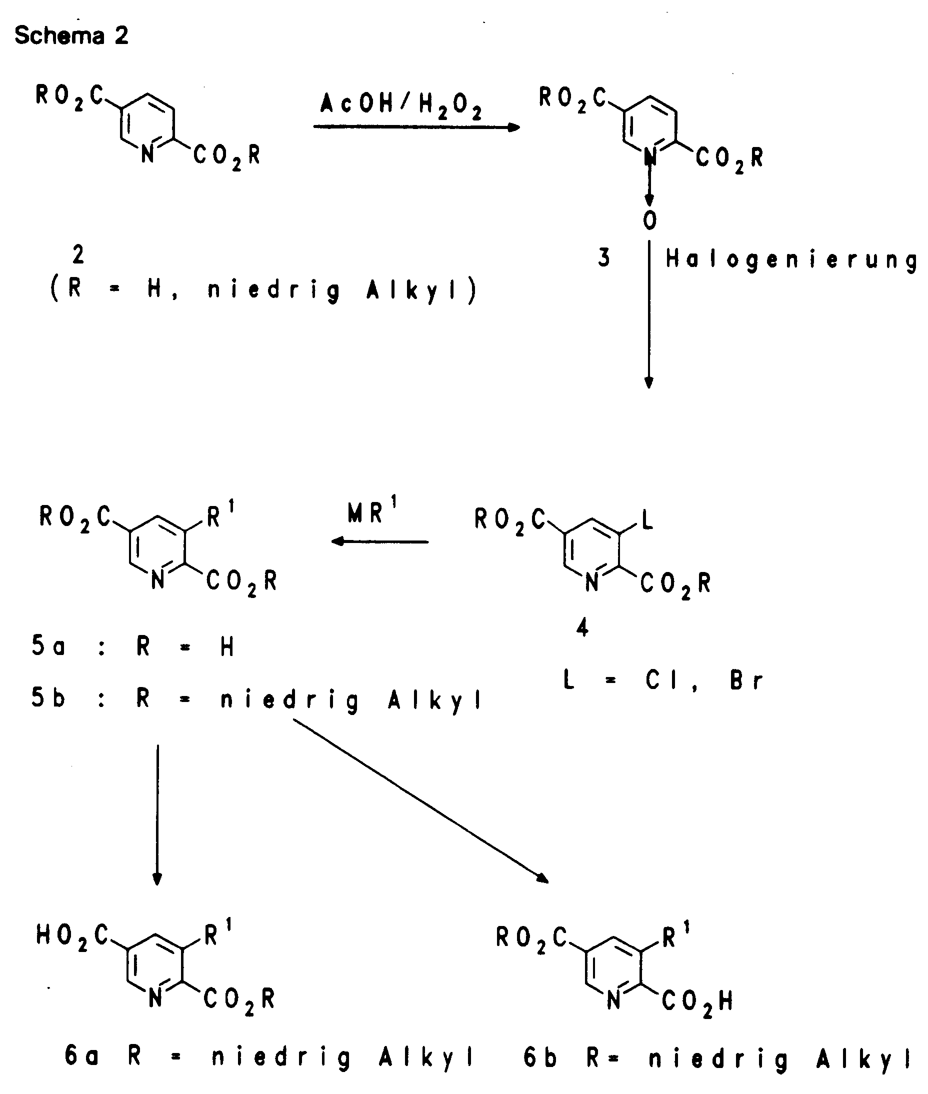

Die 3-substituierten 5-Carboxypyridin-2-carbonsäureester der Formel 6a und 6b werden aus den Pyridin-2,5-dicarbonsäurediestern der Formel 2 hergestellt.

Die Oxidation der Pyridin-2,5-dicarboxylate der Formel 2 ist in J. Chem. Soc. Perkin Trans. 2, 1978, 34-38 und in J. Org. Chem. 25 (1960) 565-568 (M.L. Peterson) beschrieben.

Die Halogenierung (Chlorierung) der Pyridin-N-oxide der Formel 3 und die Reaktion des 3-Chlorpyridin-2,5-dicarbonsäurediesters (Formel 4) mit Alkoholaten MR¹ (M = Metall; z.B. Alkali, Erdalkali) kann in Analogie zu dem in der Patentschrift CH 658 651 (LONZA) beschriebenen Verfahren durchgeführt werden.

Aus substituierten Pyridin-2,5-dicarbonsäuren (siehe CA: Vol. 68, 1968, 68840 h) können unter Veresterungsbedingungen die Pyridin-2-carbonsäureester-5-carboxylate der Formel 6a und 6b hergestellt werden. Geeignete Bedingungen sind z.B. die Veresterung mit Methanol in Gegenwart von Schwefelsäure, wobei die Reaktionszeit so zu wählen ist, daß die vollständige Veresterung zum Diesterprodukt nur untergeordnet stattfindet, bzw. die Diesterprodukte als Nebenprodukte abgetrennt werden können.

Die erfindungsgemäßen Verbindungen der Formel I besitzen wertvolle pharmakologische Eigenschaften und zeigen insbesondere antifibrotische Wirksamkeit.

Die antifibrotische Wirkung kann im Modell der Tetrachlorkohlenstoff-induzierten Leberfibrose bestimmt werden. Dazu werden Ratten mit CCl₄ (1 ml/kg) - gelöst in Olivenöl - zweimal wöchentlich behandelt. Die Prüfsubstanz wird täglich, gegebenenfalls sogar zweimal täglich per os oder intraperitoneal - gelöst in einem geeigneten verträglichen Lösungsmittel - verabreicht. Das Ausmaß der Leberfibrose wird histologisch bestimmt und der Anteil Kollagen in der Leber per Hydroxyprolinbestimmung - wie bei Kivirikko et al. (Anal. Biochem. 19, 249 f. (1967)) beschrieben- analysiert. Die Aktivität der Fibrogenese kann durch radioimmunologische Bestimmung von Kollagenfragmenten und Prokollagenpeptiden im Serum bestimmt werden. Die erfindungsgemäßen Verbindungen sind in diesem Modell in Konzentrationen von 1 - 100 mg/kg wirksam.

Die Aktivität der Fibrogenese kann durch radioimmunologische Bestimmung des N-terminalen Propeptids des Kollagens Typ-III oder der N- bzw. C-terminalen Quervernetzungsdomäne des Kollagens-Typ-IV (7s-Kollagen bzw. Typ-IV-Kollagen NC₁) im Serum bestimmt werden.

Zu diesem Zweck wurden die Hydroxyprolin-, Prokollagen-III-Peptid-, 7s-Kollagen- und Typ-IV-Kollagen-NC-Konzentrationen in der Leber von
a) unbehandelten Ratten (Kontrolle)
b) Ratten, denen Tetrachlorkohlenstoff verabreicht wurde (CCl₄-Kontrolle)
c) Ratten, denen zunächst CCl₄ und anschließend eine erfindungsgemäße Verbindung verabreicht wurde
   gemessen (diese Testmethode wird beschrieben von Rouiller, C., experimental toxic injury of the liver; in The Liver, C. Rouiller, Vol. 2, 5. 335-476, New York, Academic Press, 1964).

Die Verbindungen der Formel I können als Medikamente in Form pharmazeutischer Präparate Verwendung finden, welche sie gegebenenfalls zusammen mit verträglichen pharmazeutischen Trägern enthalten. Die Verbindungen können als Heilmittel, z.B. in Form pharmazeutischer Präparate Verwendung finden, welche diese Verbindungen in Mischung mit einem für die enterale, perkutane oder parenterale Applikation geeigneten pharmazeutischen, organischen oder anorganischen Träger, wie z.B. Wasser, Gummi arabicum, Gelatine, Milchzucker, Stärke, Magnesiumstearat, Talk, pflanzliche Öle, Polyalkylenglykole, Vaseline usw. enthalten.

Sie können zu diesem Zweck oral in Dosen von 0,1 - 25 mg/kg/Tag, vorzugsweise 1 - 5 mg/kg/Tag oder parenteral in Dosen von 0,01 - 5 mg/kg/Tag, vorzugsweise 0,01 - 2,5 mg/kg/Tag, insbesondere 0,5 - 1,0 mg/kg/Tag, appliziert werden. Die Dosierung kann in schweren Fällen auch erhöht werden. In vielen Fällen genügen jedoch auch geringere Dosen. Diese Angaben beziehen sich auf einen Erwachsenen von etwa 75 kg Gewicht.

Die Erfindung umfaßt weiterhin die Verwendung der erfindungsgemäßen Verbindungen bei der Herstellung von Arzneimitteln, die zur Behandlung und Prophylaxe der vorstehend genannten Stoffwechselstörungen eingesetzt werden.

Ein weiterer Gegenstand der Erfindung sind Arzneimitel, die ein oder mehrere erfindungsgemäße Verbindungen der Formel I und/oder deren physiologisch verträgliche Salze enthalten.

Die Arzneimittel werden nach an sich bekannten, dem Fachmann geläufigen Verfahren hergestellt. Als Arzneimittel werden die erfindungsgemäßen pharmakologisch wirksamen Verbindungen (= Wirkstoff) entweder als solche oder vorzugsweise in Kombination mit geeigneten pharmazeutischen Hilfs- oder Trägerstoffen in Form von Tabletten, Dragees, Kapseln, Suppositorien, Emulsionen, Suspensionen oder Lösungen eingesetzt, wobei der Wirkstoffgehalt bis etwa 95 %, vorteilhafterweise zwischen 10 und 75 % beträgt.

Geeignete Hilfs- bzw. Trägestoffe für die gewünschte Arzneimittelformulierung sind beispielsweise neben Lösemitteln, Gelbildner, Suppositoriengrundlagen, Tabletten-Hilfsstoffen und anderen Wirkstoffträgern auch Antioxidantien, Dispergiermittel, Emulgatoren, Entschäumer, Geschmachskorrigentien, Konservierungsmittel, Lösungsvermittler oder Farbstoffe.

Die folgenden Beispiele sollen die Erfindung erläutern.

### Beispiel 1

### N-((Ethoxycarbonyl)methyl)-3-methoxy-5-[((phenylsulfonyl)amino)carbonyl]pyridin-2-carbonsäureamid

a) 5-Methoxycarbonylpyridin-2-carbonsäure-1-oxid (vgl. J. Org. Chem. 25 (1960) 565)
   12 g (60 mMol) Pyridin-2,5-dicarbonsäure-dimethylester wurden in 30 ml Eisessig suspendiert und bei 20°C unter Rühren mit 13 ml Wasserstoffperoxid (35 %) versetzt. Unter Rühren wurde sodann auf 100°C (Innentemperatur) erwärmt, wobei sich bei 50°C eine klare Lösung bildete. Nachdem 90 min bei 100°C gerührt worden war, ließ man auf 20°C abkühlen, saugte die kristalline Fällung ab, wusch mit Wasser und nach dem Trocknen erhielt man 7,5 g Produkt, Fp. 160°C (Zers.)
b) 3-Chlorpyridin-2,5-dicarbonsäure-dimethylester
   Es wurden 17 ml Thionylchlorid, 35 ml wasserfreies Chloroform und 1,5 ml N,N-Dimethylformamid unter Rühren auf 60°C erwärmt und bei dieser Temperatur portionsweise mit 7,5 g des vorstehenden Produkts versetzt. Dann wurde weitere 60 min bei 60°C gerührt, das Lösungsmittel und überschüssiges Reagenz nach dem Abkühlen im Vakuum abdestilliert, der Rückstand mit Dichlormethan versetzt, der N,N-Dimethylformamid x HCl-Komplex abgesaugt und mit Dichlormethan gewaschen. Zur Mutterlauge gab man unter Kühlung ca. 15 ml Triethylamin und 10 ml Methanol und rührte 30 min. Nach dem Eindampfen im Vakuum wurde der Rückstand in 50 ml Wasser gelöst, 3 x mit Dichlormethan extrahiet, die organische Phase getrocknet, eingeengt und der Rückstand mit n-Heptan und n-Heptan:Ethylacetat (3:1) an Kieselgel chromatographiert. Aus entsprechenden Fraktionen wurden 5,3 g Produkt mit Petrolether zur Kristallisation gebracht, Fp. 36-38°C. c) 3-Methoxypyridin-2,5-dicarbonsäure
   53 g (0,231 mol) des vorstehenden Diesters wurden in 500 ml Methanol gelöst und unter Rühren bei 20°C mit 150 ml (0,81 mol) Natriummethylat-Lösung (30 % in Methanol) versetzt, wobei die Temperatur auf 30°C anstieg. Man erhitzte 4,5 h unter Rückfluß, versetzte bei 20°C mit 300 ml Wasser und rührte 30 min bei 35°C. Das überschüssige Methanol wurde im Vakuum abdestilliert, die wäßrige Phase unter Kühlung mit halbkonzentrierter wäßriger Salzsäure auf pH 2 gebracht, das farblose kristalline Produkt abgesaugt und getrocknet. Man erhielt 49 g, Fp. 185 ° (Gasentwicklung); 255°C (Zers.)
d) 5-Carboxy-3-methoxypyridin-2-carbonsäuremethylester
   10 g (50,7 mmol) der vorstehenden Pyridindicarbonsäure wurden in 150 ml wasserfreiem Methanol suspendiert, mit 2 ml konzentrierter Schwefelsäure versetzt und 3 h rückfließend erhitzt. Dann wurde die Hälfte des Methanols im Vakuum abdestilliert, der Rückstand in 400 ml Eiswasser eingetragen, der kristalline Rückstand abgesaugt, mit Wasser gewaschen, der Rückstand in 150 ml gesättigter wäßriger Na-bicarbonat-Lösung gelöst, zweimal mit je 80 ml Dichlormethan extrahiert, die Bicarbonat-Phase unter Kühlung mit halbkonzentrierter wäßriger Salzsäure auf pH 1 gebracht, das ausgefallene Produkt abgesaugt und getrocknet. Man erhielt 5 g farblose, kristalline Substanz, Fp. 196-197°C. Aus der Dichlormethan-Phase wurden 1,7 g Dimethylester erhalten, Fp. 53-55°C (aus Petrolether).
e) 3-Methoxy-5-[((phenylsulfonyl)amino)carbonyl]pyridin-2-carbonsäuremethylester
   2,4 g (15 mmol) Benzolsulfonsäureamid wurden in 80 ml wasserfreiem Tetrahydrofuran suspendiert, bei 20°C unter Rühren mit 1,85 g (16,5 mmol) Kalium-tert.Butylat versetzt und 30 min bei 50-60°C gerührt (Lösung A). In einem zweiten Kolben wurden 3,2 g (15 mmol) der vorstehenden Pyridincarbonsäure in 80 ml wasserfreiem Tetrahydrofuran gelöst, bei 20°C unter Rühren 2,7 g (16,5 mmol) N,N'-Carbonyldiimidazol zugegeben, 30 min bei 60°C gerührt und die abgekühlte Lösung sodann zur Lösung A getropft. Man rührte 1 h bei 60°C, engte nach dem Abkühlen im Vakumm ein, versetzte den Rückstand mit 200 ml gesättigter, wäßriger Na-bicarbonat-Lösung und extrahierte mit Dichlormethan. Die Bicarbonat-Phase wurde mit halbkonzentrierter wäßriger Salzsäure auf pH 3 gebracht und viermal mit Dichlormethan extrahiert. Nach dem Trocknen wurde Dichlormethan abdestilliert und der Rückstand mit Diethylether/Ethylacetat zur Kristallisation gebracht. Man erhielt 4,0 g Produkt, Fp. 160-163°C (ab 150°C Sintern).
f) 3-Methoxy-5-[((phenylsulfonyl)amino)carbonyl]pyridin-2-carbonsäure
   4,0 g (11,4 mmol) des vorstehenden Esters wurden bei 20°C unter Rühren in 150 ml 1,5 N methanolische Natronlauge eingetragen. Nach dem kurzzeitig eine klare Lösung entstand, fiel das Na-Salz aus. Man gab 20 ml Wasser hinzu, rührte 30 min nach, engte im Vakuum ein, löste den Rückstand in 100 ml Wasser, extrahierte mit Dichlormethan, brachte die wäßrige Phase mit konzentrierter wäßriger Salzsäure auf pH 1 und saugte 3,7 g Produkt als kristalline Fällung ab, Fp. 176-178°C.
g)
   Die Titelverbindung wurde erhalten, indem 3,7 g (10 mmol) der vorstehenden Carbonsäure in 300 ml Dichlormethan suspendiert wurde, unter Rühren nacheinander mit 1,4 g (10 mmol) Glycinethylester-Hydrochlorid, 3,9 ml (30 mmol) N-Ethylmorpholin, 1,5 g (11 mmol) 1-Hydroxy-1H-benztriazol und 4,2 g (10 mmol) N-Cyclohexyl-N'-(2-morpholinoethyl)-carbodiimid-methyl-p-toluolsulfonat versetzt und 2 Tage bei 20°C gerührt wurde. Dann wurde vom Ungelöstem abgesaugt, mit Wasser, sodann mit 1 N wäßriger Salzsäure extrahiert, die organische Phase getrocknet, eingeengt und der Rückstand mit Diethylether zur Kristallisation gebracht. Man erhielt 1,5 g Produkt, Fp. ab 100°C unter Schäumen. Aus der wäßrigen Phase wurde nach Ansäuern mit Salzsäure auf pH 3 und Extraktion mit Dichlormethan, Trocknen und Einengen weitere 1,6 g Produkt isoliert.

### Beispiel 2

### N-Ethoxycarbonylmethyl-5-[((4-(((2-(4-fluorphenyl)ethyl)amino)carbonyl)phenylsulfonyl)amino)carbonyl]-3-methoxypyridin-2-carbonsäure-glycylamid

### Beispiel 3

### N-Ethoxycarbonylmethyl-5-[((4-(((2-(4-fluorphenyl)ethyl)amino)carbonyl)phenylsulfonyl)amino)carbonyl]-3-(2-methyl-1-propyloxy)pyridin-2-carbonsäure-glycylamid

### Beispiel 4

### N-Ethoxycarbonylmethyl-5-[((4-(((2-(4-fluorphenyl)ethyl)amino)carbonyl)phenylsulfonyl)amino)carbonyl]-3-hydroxypyridin-2-carbonsäureamid

### Beispiel 5

### 5-[((4-((Cylcohexylamino)carbonyl)phenylsulfonyl)amino)carbonyl]-3-methoxypyridin-2-carbonsäure-N-((ethoxycarbonyl)methyl)amid

a) 5-[((4-((Cyclohexylamino)carbonyl)phenylsulfonyl)amino)carbonyl]-3-methoxypyridin-2-carbonsäure
   4,2 g (20 mmol) 5-Carboxy-3-methoxypyridin-2-carbonsäuremethylester (vgl. Beispiel 1d)) wurden analog Beispiel 1e) mit 5,6 g (20 mmol) 4-((Cyclohexylamino)carbonyl)benzolsulfonamid umgesetzt und das Produkt anschließend verseift. Man erhielt 7 g Produkt, Fp. ab 235°C (sintern bei 205°C, aus wäßriger Salzsäure).
b) Die Titelverbindung wurde analog Beispiel 1g) aus 1,85 g (4 mmol) der vorstehenden Verbindung erhalten; 1,3 g Produkt, Fp. 225°C (unter Schäumen, sintern bei 185°C, aus Diisopropylether).

### Beispiel 6

### 5-[((4-((Cylcohexylamino)carbonyl)phenylsulfonyl)amino)carbonyl]-3-(2-methyl-1-propyloxy)pyridin-2-carbonsäure-N-((ethoxycarbonyl)methyl)amid

a) 3-(2-Methyl-1-propyloxy)-pyridin-2,5-dicarbonsäure
   Analog Beispiel 1c) wurden 3,5 g (146 mmol) Natrium in 350 ml 2-Methyl-1-propanol (iso-Butylalkohol) gelöst und unter Rühren bei 20°C mit 13,7 g (55 mmol) 3-Chlorpyridin-2,5-dicarbonsäuredimethylester (wie in Beispiel 1b) hergestellt) versetzt. Dann wurde 90 Minuten bei 80°C gerührt, nach dem Abkühlen im Vakuum eingeengt, der Rückstand in 200 ml 1 N methanolische NaOH aufgenommen und bei 20°C gerührt. Nach 15 Minuten trübte sich die Lösung. Man setzte Wasser hinzu bis eine klare Lösung entstand, rührte 1 Stunde, engte im Vakuum ein, säuerte die wäßrige Lösung mit wäßriger Salzsäure an, saugte das kristalline Produkt ab, wusch, trocknete und erhielt 10,6 g Dicarbonsäure, Fp. 192°C (Zers.).
b) 5-Carboxy-3-(2-methyl-1-propyloxy)pyridin-2-carbonsäuremethylester wurden analog Beispiel 1d) aus der obigen Dicarbonsäure durch Veresterung in Methanol/Schwefelsäure erhalten, Fp. 158°-160°C (aus wäßriger Salzsäure).
c) 5-[((4-((Cylcohexylamino)carbonyl)phenylsulfonyl)amino)carbonyl]-3-(2-methyl-1-propyloxy)pyridin-2-carbonsäuremethylester wurde aus 1,5 g (6 mmol) des vorstehenden Produkts mit N,N'-Carbonyldiimidazol und 4-((Cyclohexylamino)carbonyl)benzolsulfonamid (Fp. 268-269°C, aus Ethanol/Wasser (1:1) und Kalium-tert. butylat erhalten, Fp. 270°C (aus Diisopropylether).
d) 5-[((4-((Cyclohexylamino)carbonyl)phenylsulfonyl)amino)carbonyl]-3-(2-methyl-1-propyloxy)pyridin-2-carbonsäure wurde durch Verseifung des obigen Esters erhalten, Fp. > 150°C (unter Schäumen, aus wäßriger Salzsäure).
e) Die Titelverbindung wurde analog Beispiel 1g) erhalten. Aus 1,55 g (3,1 mmol) der vorstehenden Carbonsäure und 0,43 g (3,1 mmol) Glycinethylester-Hydrochlorid wurden 1,46 g Produkt gewonnen, Fp. > 280°C (unter Gasentwicklung, sintern bei 135°C, aus Diethylether).

### Beispiel 7

### N-Ethoxycarbonylmethyl-5-[((4-((cylcohexylamino)carbonyl)phenylsulfonyl)amino)carbonyl]-3-hydroxypyridin-2-carbonsäureamid

### Beispiel 8

### N-Ethoxycarbonylmethyl-5-[((4-(N,N-diethylaminocarbonyl)phenylsulfonyl)amino)carbonyl]-3-methoxypyridin-2-carbonsäureamid

### Beispiel 9

### N-Ethoxycarbonylmethyl-5-[((4-(N,N-diethylaminocarbonyl)phenylsulfonyl)amino)carbonyl]-3-(2-methyl-1-propyloxy)pyridin-2-carbonsäureamid

### Beispiel 10

### N-Ethoxycarbonylmethyl-5-[((4-(N,N-diethylaminocarbonyl)phenylsulfonyl)amino)carbonyl]-3-hydroxypyridin-2-carbonsäureamid

### Beispiel 11

### N-Ethoxycarbonylmethyl-5-[((4-(N,N-dipropylaminocarbonyl)phenylsulfonyl)amino)carbonyl]-3-methoxypyridin-2-carbonsäureamid

### Beispiel 12

### N-Ethoxycarbonylmethyl-5-[((4-(4,4-dibutylaminocarbonyl)phenylsulfonyl)amino)carbonyl]-3-methoxypyridin-2-carbonsäureamid

### Beispiel 13

### N-Ethoxycarbonylmethyl-5-[((4-(2-((2-chlor-5-methoxybenzoyl)amino)ethyl)phenylsulfonyl)amino)-carbonyl]-3-methoxypyridin-2-carbonsäureamid

### Beispiel 14

### N-Ethoxycarbonylmethyl-5-[((4-(2-((2-chlor-5-methoxybenzoyl)amino)ethyl)phenylsulfonyl)amino)-carbonyl]-3-(2-methyl-1-propyloxy)pyridin-2-carbonsäureamid

### Beispiel 15

### N-Ethoxycarbonylmethyl-5-[((4-(2-((2-chlor-5-methoxybenzoyl)amino)ethyl)phenylsulfonyl)amino)-carbonyl]-3-hydroxypyridin-2-carbonsäureamid

### Beispiel 16

### N-Ethoxycarbonylmethyl-5-[((4-n-butyloxyphenyl)sulfonyl)amino)carbonyl]-3-methoxypyridin-2-carbonsäureamid

### Beispiel 17

### N-Ethoxycarbonylmethyl-5-[((n-butylsulfonyl)amino)carbonyl]-3-methoxypyridin-2-carbonsäureamid

### Beispiel 18

### N-Ethoxycarbonylmethyl-5-[((4-fluorphenyl)sulfonyl)amino)carbonyl]-3-methoxypyridin-2-carbonsäureamid

### Beispiel 19

### N-Ethoxycarbonylmethyl-3-methoxy-5-[((4-propylphenyl)sulfonyl)amino)carbonyl]pyridin-2-carbonsäureamid

### Beispiel 20

### N-Ethoxycarbonylmethyl-3-hydroxy-5-[((4-propylphenyl)sulfonyl)amino)carbonyl]pyridin-2-carbonsäureamid

### Beispiel 21

### N-Ethoxycarbonylmethyl-5-[((n-butylsulfonyl)amino)carbonyl]-3-(3-methyl-1-butyloxy)pyridin-2-carbonsäureamid

### Beispiel 22

### N-Ethoxycarbonylmethyl-5-[((benzylsulfonyl)amino)carbonyl]-3-methoxypyridin-2-carbonsäureamid

### Beispiel 23

### N-(3-Pentyl)oxycarbonylmethyl-5-[((4-(N,N-diethylaminocarbonyl)phenylsulfonyl)amino)carbonyl]-3-methoxypyridin-2-carbonsäureamid

### Beispiel 24

### N-Benzyloxycarbonylmethyl-5-[((4-((cyclohexylamino)carbonyl)phenylsulfonyl)amino)carbonyl]-3-methoxypyridin-2-carbonsäureamid

### Beispiel 25

### 5-[((1-Decylsulfonyl)amino)carbonyl]-3-methoxypyridin-2-carbonsäure-N-((ethoxycarbonyl)methyl)amid

a) 5-[((1-Decylsulfonyl)amino)carbonyl]-3-methoxypyridin-2-carbonsäure
   2,1 g (10 mmol) 5-Carboxy-3-methoxypyridin-2-carbonsäuremethylester (vgl. Beispiel 1d)) wurden analog wie in Beispiel 1e) beschrieben mit N,N'-Carbonyldiimidazol und 1-Decylsulfonsäureamid/Kalium-tert.butylat umgesetzt und der so erhaltene Pyridin-2-carbonsäuremethylester mit 1 N methanolischer Natronlauge verseift. Nachdem die wäßrige Lösung unter Kühlung angesäuert wurde, erhielt man 1,4 g Produkt, Fp. 145°C (unter Zers.).
b) 1 g (2,5 mmol) der vorstehenden Pyridin-2-carbonsäure wurden analog wie in Beispiel 1g) beschrieben mit Glycinethylester-Hydrochlorid kondensiert. Nach Aufarbeitung wurde der Rückstand mit Diisopropylether zur Kristallisation gebracht. Man erhielt 1,1 g der Titelverbindung, Fp. 70°C.

### Beispiel 26

### 5-[((1-Hexadecylsulfonyl)amino)carbonyl]-3-methoxypyridin-2-carbonsäure-N-((ethoxycarbonyl)methyl)amid

a) 5-[((1-Hexadecylsulfonyl)amino)carbonyl]-3-methoxypyridin-2-carbonsäuremethylester wurde analog Beispiel 1e) aus 3,2 g (15 mmol) 5-Carboxy-3-methoxypyridin-2-carbonsäure-methylester (vgl. 1d)) / N,N'-carbonyldiimidazol und 4,8 g (15 mmol) 1-Hexadecylsulfonsäureamid (Fp. 98-100°C, aus wäßriger Salzsäure)/Kalium-tert. butylat erhalten, 6,1 g Produkt, Fp. 75-78°C (aus wäßriger Salzsäure).
b) 5-[((1-Hexadecylsulfonyl)amino)carbonyl]-3-methoxypyridin-2-carbonsäure wurde durch Verseifung des obigen Esters erhalten, Fp. 152°C (unter Zers., aus Ethylacetat).
c) Die Titelverbindung wurde analog Beispiel 1g) erhalten, Fp. 127-130°C (unter Schäumen, aus Diisopropylether).

### Beispiel 27

### 5-[((1-Decylsulfonyl)amino)carbonyl]-3-methoxypyridin-2-carbonsäure-N-(((1-butyloxy)carbonyl)methyl)amid

Die Titelverbindung wurde aus 0,5 g (1,25 mmol) 5-[((1-Decylsulfonyl)amino)carbonyl]-3-methoxypyridin-2-carbonsäure (vgl. Beispiel 25a)) und 0,46 g (1,5 mmol) Glycin-(1-butyl)ester-Tosylat (hergestellt aus Glycin, 1 -Butanol, p-Tos·OH mit Toluol am Wasserabscheider) hergestellt. Man erhielt 0,31 g Produkt, Fp. 82-85°C (aus Diisopropylether/Ethylacetat (2:1)).

### Beispiel 28

### 5-[((4-((Cyclohexylamino)carbonyl)phenylsulfonyl)amino)carbonyl]-3-methoxypyridin-2-carbonsäure-N-(((1-octyloxy)carbonyl)methyl)amid

Die Titelverbindung wurde aus 5-[((4-((Cyclohexylamino)carbonyl)phenylsulfonyl)amino)carbonyl]-3-methoxypyridin-2-carbonsäure (vgl. Beispiel 5a)) und Glycin-(1-octyl)ester-tosylat (hergestellt aus Glycin, 1-Octanol, p-Tos·OH mit Toluol am Wasserabscheider) analog Beispiel 5b) erhalten, Fp. ab 220°C (Schäumen, sintern ab 160°C, aus Diethylether).

### Beispiel 29

### 3-Methoxy-5-[((phenylsulfonyl)amino)carbonyl]pyridin-2-carbonsäure-N-(((1-hexadecyloxy)carbonyl)methyl)amid

Die Titelverbindung wurde erhalten, indem 1,35 g (4 mmol) 3-Methoxy-5-[((phenylsulfonyl)amino)carbonyl]pyridin-2-carbonsäure(vgl. Beispiel 1f)) analog Zu Beispiel 1 g) mit 1,9 g (4 mmol) Glycin-(1-hexadecyl)ester-tosylat umgesetzt wurden. Man erhielt 1,9 g farblos kristallines Produkt aus Diisoproylether, Fp. 133-135°C.

## Patentansprüche

1. Sulfonamidocarbonylpyridin-2-carbonsäureesteramide sowie ihre Pyridin-N-oxide der allgemeinen Formel I, in der 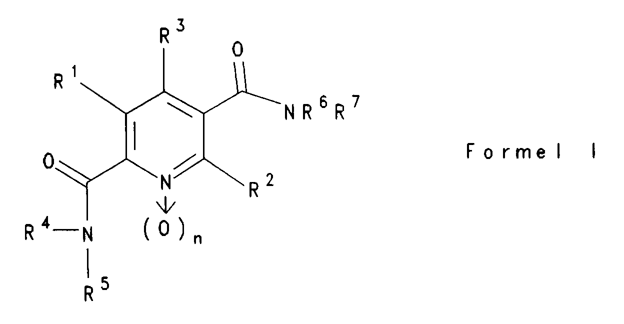
R¹ Hydroxy, (C₁-C₆)-Alkoxy,
R² und R³ Wasserstoff,
R⁶ Wasserstoff oder ein 1 oder 2-wertiges physiologisch verwendbares Kation, insbesondere Na⊕, K⊕, Mg²⊕ oder Ca²⊕ oder ein Ammoniumion, insbesondere H₃N⊕C(CH₂OH)₃
R⁷ einen Rest der Formel II, ausgenommen -SO₂H, bedeutet
-Y-[C-U]ᵣ-D-W (II),
in welchem
Y -SO₂-,
C eine Bindung oder
(C₁-C₄)-Alkandiyl,
U eine Bindung,
r 1 ist,
D eine Bindung oder Wasserstoff,
W einen Phenylrest bedeutet, wobei W einfach oder zweifach substituiert sein kann durch Fluor, Chlor, (C₁-C₆)-Alkyl, (C₁-C₆)-Alkoxy,
und W zusätzlich substituiert ist durch Phenyl, Phenoxy, -O-[CH₂]_{X}C_{f}H_{(2f+1-g)}F_{g},Carbamoyl, N-(C₁-C₁₀)-Alkylcarbamoyl, N,N-Di-(C₁-C₈)-alkylcarbamoyl, N-(C₃-C₈)-Cycloalkylcarbamoyl, N-Phenylcarbamoyl, N-(C₇-C₁₁)-Phenylalkylcarbamoyl, N-((C₁-C₄)-Alkoxy-(C₁-C₄)-alkyl)-carbamoyl, N-Phenoxy-(C₁-C₄)alkyl)carbamoyl,
(C₁-C₁₀)-Alkanoylamino-(C₁-C₂)-alkyl, (C₃-C₈)-Cycloalkanoylamino-(C₁-C₂)-alkyl, Benzoylamino-(C₁-C₂)-alkyl oder (C₇-C₁₁)-Phenylalkanoylamino-(C₁-C₂)-alkyl,
wobei die Reste, die einen Arylrest enthalten, ihrerseits am Aryl substituiert sein können mit 1, 2, 3, 4 oder 5 gleichen oder verschiedenen Substituenten aus der Reihe Hydroxy, Carboxyl, (C₁-C₄)-Alkoxy, Phenoxy, Benzyloxy, Fluor, Chlor ,Trifluormetyl
Carbamoyl, N-(C₁-C₆)-Alkylcarbamoyl, N,N-Di-(C₁-C₆)-alkylcarbamoyl, N-(C₃-C₈)-Cycloalkylcarbamoyl, N-Phenylcarbamoyl, N-(C₇-C₁₁)-Phenalkylcarbamoyl,
R⁴ Wasserstoff,
R⁵ eine Methyl-Gruppe bedeutet, die mit (C₁-C₈)-Alkoxycarbonyl, (C₅-C₆)-Cycloalkoxycarbonyl oder Benzyloxycarbonyl substituiert ist
n 0,
f 1 bis 5,
g 0,1 bis (2f + 1) und
x 0 oder 1
bedeuten.

2. Verfahren zur Herstellung von Verbindungen nach Anspruch 1, dadurch gekennzeichnet, daß man Pyridin-2-carbonsäureestercarboxylate der Formel 7 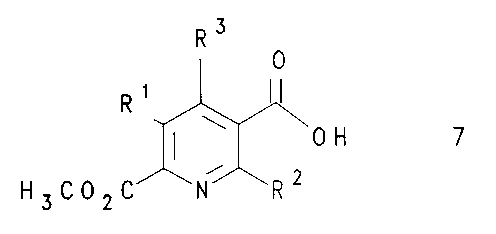 mit einem Sulfonsäureamidderivat der Formel 8
R⁶HN-R⁷ 8
und die hieraus entstehenden Verbindungen mit einem Amin der Formel 5
HNR⁴R⁵ 5
zu den Verbindungen der Formel Ia' umsetzt, 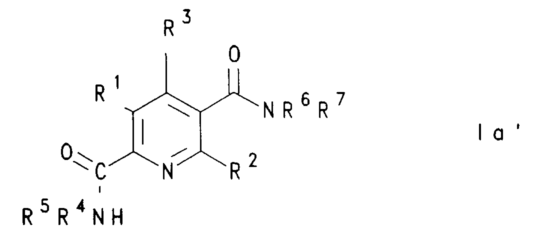 wobei man ggf. eine Oxidation zum Pyridin-N-oxid gemäß Formel Ib' anschließt

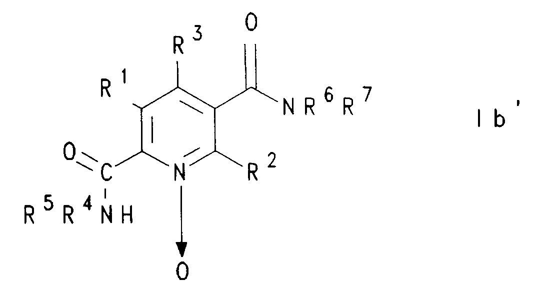

3. Verbindungen nach Anspruch 1 zur Anwendung gegen fibrotische Erkrankungen.

4. Verbindungen nach Anspruch 1 zur Anwendung als Fibrosuppressiva.

5. Verbindungen nach Anspruch 1 zur Anwendung als Inhibitoren der Prolylhydroxylaxe.

6. Arzneimittel, enthaltend mindestens eine Verbindung gemäß Anspruch 1 und ggf. einen pharmazeutisch verträglichen Träger.

7. Verbindungen gemäß Anspruch 1 zur Behandlung von Störungen des Stoffwechsels von Collagen und collagenähnlichen Stoffen.

8. Verbindungen gemäß Anspruch 1 zur Behandlung von fibrotischen Erkrankungen.

9. Verfahren zur Herstellung von Arzneimitteln zur Behandlung von fibrotischen Erkrankungen, dadurch gekennzeichnet, daß das Arzneimittel eine Verbindung gemäß Anspruch 1 enthält.

## Claims

1. A sulfonamidocarbonylpyridine-2-carboxamide or its pyridine N-oxide of the formula I 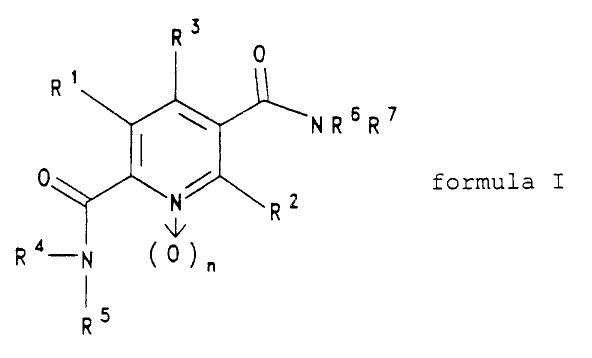 in which
R¹ is hydroxyl or (C₁-C₆)-alkoxy,
R² and R³ are hydrogen,
R⁶ is hydrogen or a 1- or 2-valent physiologically utilizable cation, in particular Na^{⊕}, K^{⊕}, Mg^{2⊕} or Ca^{2⊕} or an ammonium ion, in particular H₃N⊕C(CH₂OH)₃,
R⁷ is a radical of the formula II, excluding -SO₂H,
-Y-[C-U]ᵣ-D-W (II),
in which
Y is -SO₂-,
C is a bond or
(C₁-C₄)-alkanediyl,
U is a bond,
r is 1,
D is a bond or hydrogen,
W is a phenyl radical, where W can be substituted once or twice by fluorine, chlorine, (C₁-C₆)-alkyl or (C₁-C₆)-alkoxy, and W is additionally substituted by phenyl, phenoxy, -O-[CH₂]_{X}C_{f}H_{(2f+1-g)}F_{g}, carbamoyl, N-(C₁-C₁₀)-alkylcarbamoyl, N,N-di-(C₁-C₈)-alkylcarbamoyl, N-(C₃-C₈)-cycloalkylcarbamoyl, N-phenylcarbamoyl, N-(C₇-C₁₁)-phenylalkyl-carbamoyl, N-((C₁-C₄)-alkoxy-(C₁-C₄)-alkyl)-carbamoyl, N-(phenoxy-(C₁-C₄)alkyl)carbamoyl,
(C₁-C₁₀)-alkanoylamino-(C₁-C₂)-alkyl, (C₃-C₈)-cycloalkanoylamino-(C₁-C₂)-alkyl, benzoylamino-(C₁-C₂)-alkyl or (C₇-C₁₁)-phenylalkanoylamino-(C₁-C₂)-alkyl,
where the radicals which contain an aryl radical can for their part be substituted on the aryl by 1, 2, 3, 4 or 5 identical or different substituents from the series hydroxyl, carboxyl, (C₁-C₄)-alkoxy, phenoxy, benzyloxy, fluorine, chlorine, trifluoromethyl,
carbamoyl, N-(C₁-C₆)-alkylcarbamoyl, N,N-di-(C₁-C₆)-alkylcarbamoyl, N-(C₃-C₈)-cycloalkylcarbamoyl, N-phenylcarbamoyl, N-(C₇-C₁₁)-phenalkylcarbamoyl,
R⁴ is hydrogen,
R⁵ is a methyl group which is substituted by (C₁-C₈)-alkoxycarbonyl, (C₅-C₆)-cycloalkoxycarbonyl or benzyloxycarbonyl,
n is 0,
f is 1 to 5,
g is 0,1 to (2f+1) and
x is 0 or 1.

2. A process for the preparation of compounds as claimed in claim 1, which comprises reacting a pyridine-2-carboxylic acid ester carboxylate of the formula 7 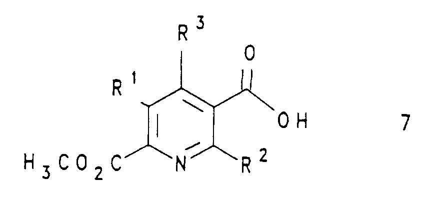 with a sulfonamide derivative of the formula 8
R⁶HN-R⁷ 8
and reacting the compound resulting therefrom with an amine of the formula 5
HNR⁴R⁵ 5
to give the compound of the formula Ia' 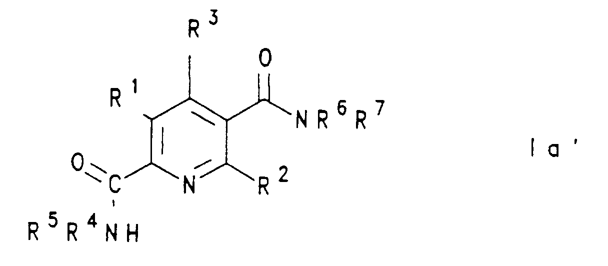 an oxidation to the pyridine N-oxide according to formula Ib' optionally following

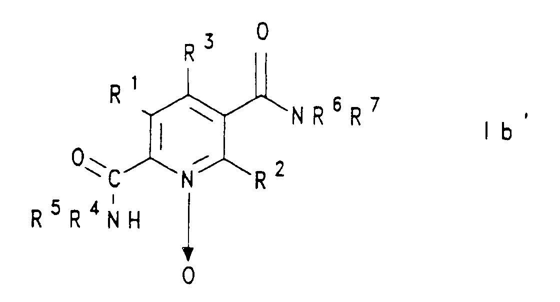

3. A compound as claimed in claim 1 for use against fibrotic disorders.

4. A compound as claimed in claim 1 for use as a fibrosuppressant.

5. A compound as claimed in claim 1 for use as an inhibitor of prolyl hydroxylase.

6. A pharmaceutical, containing at least one compound as claimed in claim 1 and optionally a pharmaceutically tolerable vehicle.

7. A compound as claimed in claim 1 for the treatment of disorders of the metabolism of collagen and collagen-like substances.

8. A compound as claimed in claim 1 for the treatment of fibrotic disorders.

9. A process for the production of pharmaceuticals for the treatment of fibrotic disorders, wherein the pharmaceutical contains a compound as claimed in claim 1.

## Revendications

1. Ester-amides d'acides sulfonamidocarbonylpyridine-2-carboxyliques ainsi que leurs N-oxydes de pyridine de formule générale I 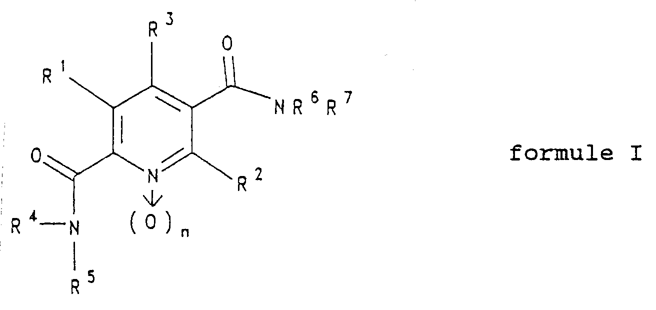 dans laquelle
R¹ représente un groupe hydroxyle ou alcoxy en C₁-C₆,
R² et R³ sont des atomes d'hydrogène,
R⁶ représente un atome d'hydrogène ou un cation mono- ou divalent physiologiquement utilisable, en particulier Na^{⊕}, K^{⊕}, Mg^{2⊕} ou Ca^{2⊕} ou un ion ammonium, en particulier H₃N^{⊕}C(CH₂OH)₃,
R⁷ représente un radical de formule II, à l'exception de -SO₂H,
-Y-[C-U]ᵣ-D-W (II)
dans laquelle
Y représente -SO₂-,
C représente une liaison ou
un radical alcanediyle en C₁-C₄
U représente une liaison,
r est égal à 1,
D représente une liaison ou un atome d'hydrogène,
W représente un radical phényle, W pouvant être une ou deux fois substitué par un ou des atomes de fluor ou de chlore ou groupes alkyle en C₁-C₆, alcoxy en C₁-C₆,
et W étant en outre substitué par un groupe phényle, phénoxy, -O-[CH₂]ₓC_{f}H_{(2f+1-g)}F_{g}, carbamoyle, N-alkyl(C₁-C₁₀)-carbamoyle, N,N-dialkyl(C₁-C₈)-carbamoyle, N-cycloalkyl(C₃-C₈)-carbamoyle, N-phénylcarbamoyle, N-phénylalkyl(C₇-C₁₁)-carbamoyle, N-[alcoxy(C₁-C₄)-alkyl(C₁-C₄)]-carbamoyle, N-[phénoxyalkyl(C₁-C₄)]-carbamoyle,
alcanoyl(C₁-C₁₀)amino-alkyle(C₁-C₂), cycloalcanoyl(C₃-C₈)amino-alkyle(C₁-C₂), benzoylamino-alkyle(C₁-C₂) ou phénylalcanoyl(C₇-C₁₁)amino-alkyle(C₁-C₂),
les radicaux qui contiennent un fragment aryle pouvant pour leur part être substitués sur le fragment aryle par 1, 2, 3, 4 ou 5 substituants identiques ou différents choisis parmi les atomes de fluor et de chlore et les groupes hydroxyle, carboxyle, alcoxy en C₁-C₄, phénoxy, benzyloxy et trifluorométhyle,
carbamoyle, N-alkyl(C₁-C₆)-carbamoyle, N,N-dialkyl(C₁-C₆)-carbamoyle, N-cycloalkyl(C₃-C₈)-carbamoyle, N-phénylcarbamoyle, N-phénylalkyl(C₇-C₁₁)-carbamoyle,
R⁴ représente un atome d'hydrogène,
R⁵ représente un radical méthyle qui est substitué par un groupe alcoxy(C₁-C₈)-carbonyle, cycloalcoxy(C₅-C₆)-carbonyle ou benzyloxycarbonyle, et
n est égal à zéro,
f va de 1 à 5,
g représente 0 ou 1 à (2f + 1) et
x est 0 ou 1.

2. Procédé pour la préparation des composés selon la revendication 1, caractérisé en ce que l'on fait réagir des esters d'acide pyridine-2-carboxylique-carboxylates de formule 7 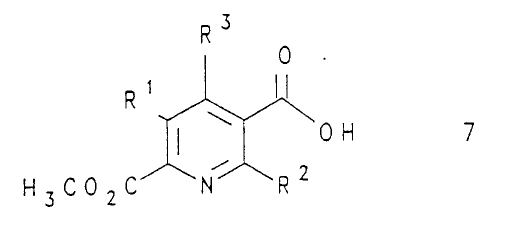 avec un dérivé de sulfonamide de formule 8
R⁸HN-R⁷ 8
et on fait réagir les composés résultants avec une amine de formule 5
R⁸NR⁴R⁵ 5
pour aboutir aux composés de formule Ia' 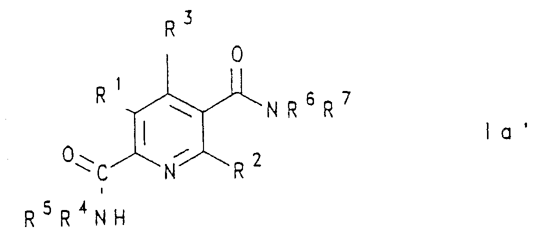 en effectuant ensuite éventuellement une oxydation en le N-oxyde de pyridine de formule Ib'

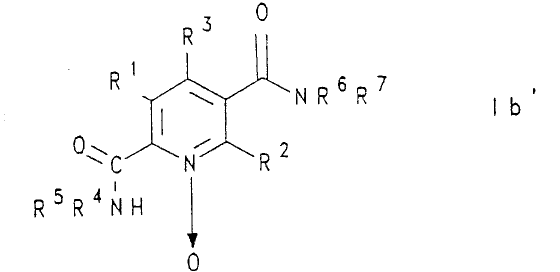

3. Composés selon la revendication 1, pour utilisation contre des maladies fibreuses.

4. Composés selon la revendication 1, pour utilisation en tant que fibrosuppresseurs.

5. Composés selon la revendication 1, pour utilisation en tant qu'inhibiteurs de la propylhydroxylase.

6. Médicament, contenant au moins un composé selon la revendication 1 et éventuellement un véhicule pharmaceutiquement acceptable.

7. Composés selon la revendication 1, pour le traitement de troubles du métabolisme du collagène et de substances de type collagène.

8. Composés selon la revendication 1, pour le traitement de maladies fibreuses.

9. Procédé pour la fabrication de médicaments destinés au traitement de maladies fibreuses, caractérisé en ce que le médicament contient un composé selon la revendication 1.
